# EUROPEAN PATENT APPLICATION

(11) **EP 4 424 226 A1**
(43) Date of publication of application: **04.09.2024**
(21) Application number: 24159475.3
(22) Date of filing: 23.02.2024
(51) Int. Cl.: A61B 1/00, A61B 5/107, G06V 10/82, G06T 7/579, G06T 7/50, A61B 1/06

(54) **MEDICAL ENDOSCOPE SYSTEM AND OPERATION METHOD THEREOF**

(30) Priority: 03.03.2023 JP 2023032566
(71) Applicant: FUJIFILM Corporation, Tokyo 106-8620 (JP)
(72) Inventor: HIGA, Katsuyuki, Kanagawa, 258-8577 (JP)
(74) Representative: Hughes, Andrea Michelle

(57) **Abstract**

A medical endoscope system and an operation method of a medical endoscope system with which a distance to a subject can be more accurately perceived are provided.

A processor device of an endoscope system functions as a pseudodistance data generation unit (74), an actual measurement distance data acquisition unit (76), and a distance data correction unit (78). The pseudodistance data generation unit (74) generates pseudodistance data obtained by estimating a distance to a subject for a plurality of locations within an imaging range. The actual measurement distance data acquisition unit (76) acquires actual measurement distance data obtained by measuring the distance to the subject for at least one location within the imaging range. The distance data correction unit (78) corrects the pseudodistance data using the actual measurement distance data.

## Description

### BACKGROUND OF THE INVENTION

### 1. Field of the Invention

The present invention relates to a medical endoscope system and an operation method thereof.

### 2. Description of the Related Art

JP2021-189822A discloses an industrial endoscope that constructs a three-dimensional structure model indicating a three-dimensional structure inside a pipe such as a gas pipe using Structure-from-Motion (SfM) and that generates data (pseudodistance data) obtained by estimating a distance to each position (subject) within a captured image using the three-dimensional structure model. However, the pseudodistance data is data obtained by estimating the distance to the subject and thus, may be different from the actual distance. Thus, in JP2021-189822A, the pseudodistance data is corrected using a radius of the pipe based on a fact that the radius of the pipe is constant or can be known in advance.

### SUMMARY OF THE INVENTION

However, while JP2021-189822A is based on an assumption that the radius of the pipe is constant or can be known in advance, the radius of the pipe is not constant or cannot be known in advance in a medical endoscope that images an inside of a body cavity such as a gastrointestinal tract as the subject. Thus, in the medical endoscope, an issue arises in that the pseudodistance data cannot be corrected and error occurs in the distance to the subject.

The present invention is conceived in view of the above background, and an object thereof is to provide a medical endoscope system and an operation method of a medical endoscope system with which a distance to a subject can be more accurately perceived.

In order to address the issue, a medical endoscope system according to an aspect of the present invention comprises an endoscope including an image sensor that acquires a captured image by imaging an inside of a body cavity as a subject, and a processor, in which the processor is configured to generate pseudodistance data obtained by estimating a distance to the subject for a plurality of locations within an imaging range of the image sensor using the captured image, acquire actual measurement distance data obtained by measuring the distance to the subject for at least one location within the imaging range, and correct the pseudodistance data using the actual measurement distance data.

The pseudodistance data may be generated using a three-dimensional structure model of the subject that is constructed from captured images of a plurality of frames captured by changing a relative positional relationship between the subject and the image sensor.

The pseudodistance data may be generated using a distance estimation model that is generated by machine learning using captured images of a plurality of frames captured by changing a relative positional relationship between the subject and the image sensor.

The actual measurement distance data may be measured using a captured image that is captured by irradiating the subject with auxiliary measurement light of which an optical axis is inclined with respect to an imaging optical axis of the image sensor.

The auxiliary measurement light may be laser light.

The subject may be a gastrointestinal tract.

The processor may be configured to, for the pseudodistance data and the actual measurement distance data of the same location, calculate a scale factor by dividing the actual measurement distance data by the pseudodistance data, and correct pseudodistance data of a location different from the same location by multiplying the pseudodistance data of the different location by the calculated scale factor.

The processor may be configured to correct pseudodistance data of all locations by multiplying all pieces of the pseudodistance data by the scale factor.

The processor may be configured to divide the subject into a plurality of regions, and correct pseudodistance data of a region including the same location by multiplying the pseudodistance data within the region including the same location by the scale factor.

The processor may be configured to, for a region for which the actual measurement distance data is not present, determine a scale factor to be used for correcting the region using a scale factor used for correcting a region for which the actual measurement distance data is present.

The processor may be configured to correct the region for which the actual measurement distance data is not present using an average value of the scale factors used for correcting the region for which the actual measurement distance data is present as the scale factor.

The processor may be configured to, for a region for which a plurality of pieces of the actual measurement distance data are present, determine a scale factor to be used for correcting the region using a scale factor of each location for which the actual measurement distance data is present.

The processor may be configured to correct the region for which the plurality of pieces of actual measurement distance data are present using an average value of the scale factors of each location for which the actual measurement distance data is present as the scale factor.

Of course, in the medical endoscope system according to the aspect of the present invention, any plurality of configurations among the above various configurations may be combined with each other.

In addition, in order to address the issue, an operation method of a medical endoscope system according to another aspect of the present invention comprises a pseudodistance data generation step of generating pseudodistance data obtained by estimating a distance to a subject for a plurality of locations within an imaging range of an image sensor of an endoscope using a captured image captured by the image sensor, an actual measurement distance data acquisition step of acquiring actual measurement distance data obtained by measuring the distance to the subject for at least one location within the imaging range, and a distance data correction step of correcting the pseudodistance data using the actual measurement distance data.

According to the present invention, the distance to the subject can be more accurately perceived.

### BRIEF DESCRIPTION OF THE DRAWINGS

Fig. 1 is a schematic diagram of an endoscope system.
Fig. 2 is a plan view illustrating a tip part of an endoscope.
Fig. 3 is a block diagram illustrating functions of the endoscope system.
Fig. 4 is a descriptive diagram illustrating a sleeve-shaped object and a protruding portion inside the sleeve-shaped object.
Fig. 5 is a descriptive diagram illustrating a captured image obtained by capturing an imaging range E1 in Fig. 4.
Fig. 6 is a descriptive diagram illustrating a captured image obtained by capturing an imaging range E2 in Fig. 4.
Fig. 7 is a descriptive diagram illustrating a captured image obtained by capturing an imaging range E3 in Fig. 4.
Fig. 8 is a flowchart illustrating a correction procedure of pseudodistance data.

### DESCRIPTION OF THE PREFERRED EMBODIMENTS

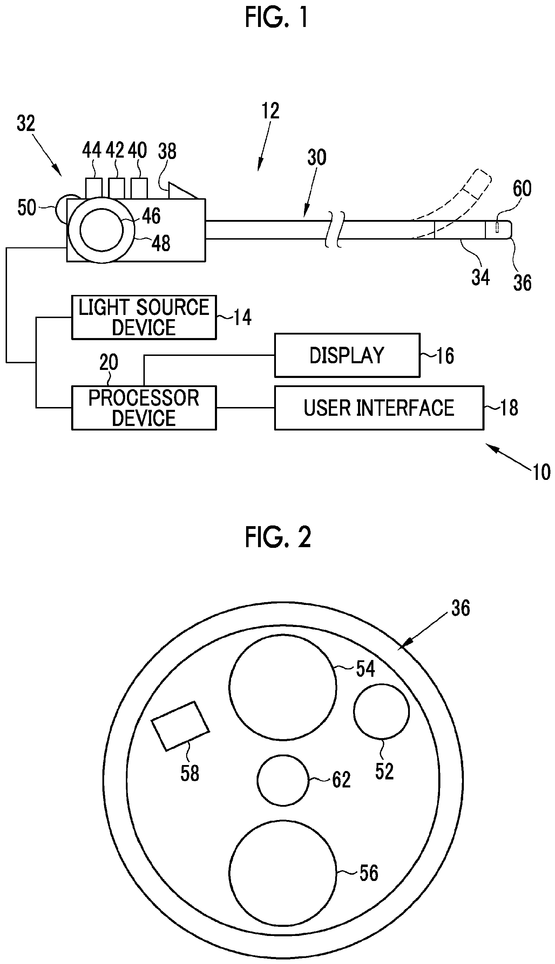

As illustrated in Fig. 1, an endoscope system 10 according to an embodiment of the present invention is a medical endoscope system used for medical purposes and includes an endoscope 12, a light source device 14, a display 16, a user interface 18, and a processor device 20 (processor).

In the endoscope system 10, the endoscope 12 is optically connected to the light source device 14 and is electrically connected to the processor device 20. In addition, in the endoscope system 10, the processor device 20 is electrically connected to each unit (the endoscope 12, the light source device 14, the display 16, and the user interface 18) of the endoscope system 10.

The endoscope 12 includes, for example, an insertion part 30 inserted into a body cavity such as a gastrointestinal tract, an operating part 32 connected to a proximal end side of the insertion part 30, and a bendable part 34 and a tip part 36 provided on a distal end side of the insertion part 30. The operating part 32 is provided with an inlet 38 of a forceps channel for inserting a treatment tool such as forceps. In addition, the operating part 32 is provided with various operating members that receive operations such as bending of the bendable part 34, zooming during subject imaging, a still image/video image capturing instruction, switching of an imaging mode, air supply, and water supply from a user. In the present embodiment, rotary dials 40, 42, and 44 subjected to a rotation operation and push buttons 46, 48, and 50 subjected to a push operation are provided as the above operating members.

As illustrated in Fig. 2, the tip part 36 is provided with an illumination window 52 for emitting illumination light, an observation window 54 for capturing reflected light of the illumination light reflected by a subject, an outlet 56 of the forceps channel, and an air supply and water supply port 58.

An image sensor 60 (refer to Fig. 1) is provided behind the observation window 54. The image sensor 60 is an image sensor such as a charge coupled device (CCD) or a complementary MOS (CMOS) that outputs a captured image as a digital image signal and images a space in front of the tip part 36 (in front of the subject). The image (captured image) captured by the image sensor 60 is input into the processor device 20.

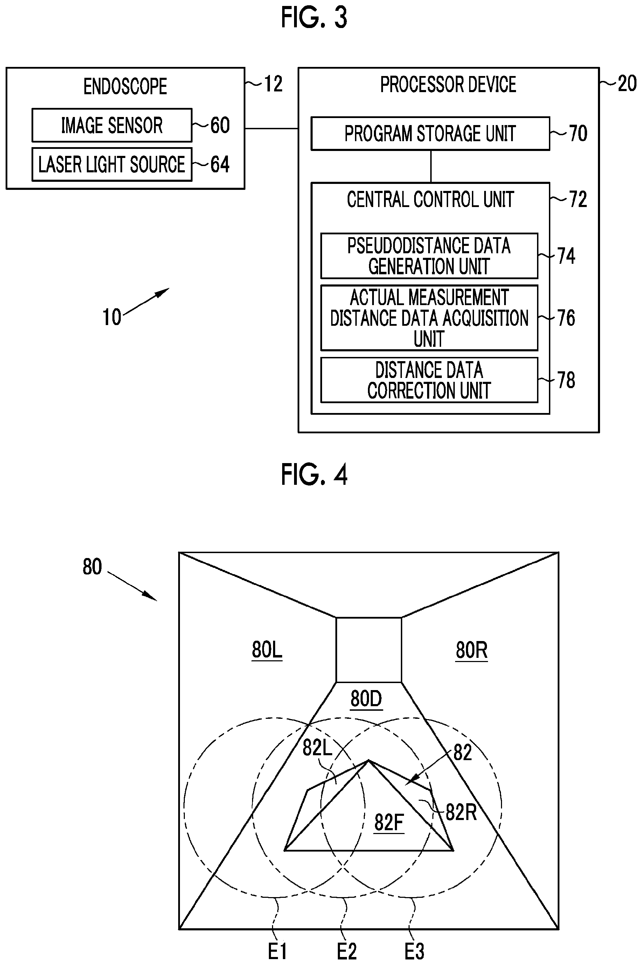

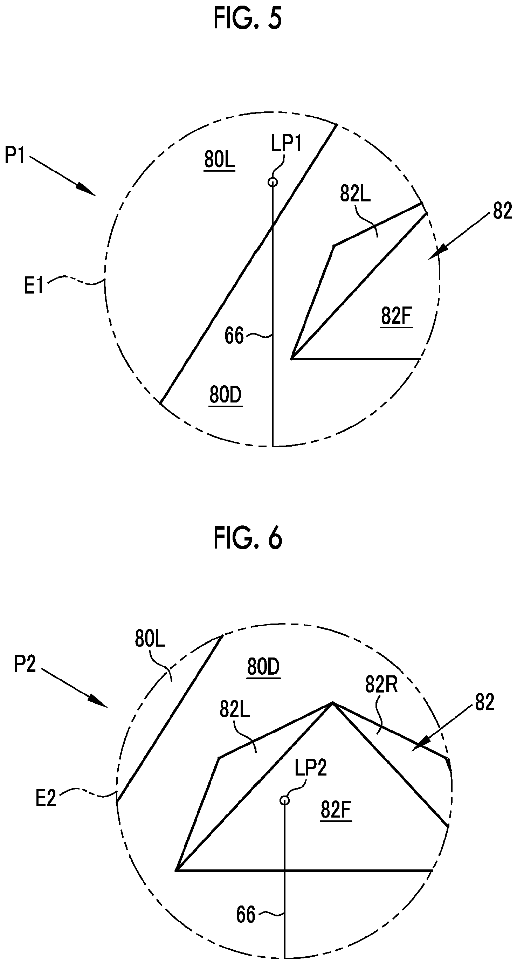

In addition, the tip part 36 is provided with an auxiliary measurement light exit window 62 for emitting auxiliary measurement light for actually measuring (measuring) a distance to the subject. The auxiliary measurement light is light of which an optical axis is inclined with respect to an imaging optical axis of the endoscope 12 (image sensor 60). In the present embodiment, the auxiliary measurement light exit window 62 is provided below the observation window 54, and laser light 66 (refer to Fig. 5, Fig. 6, and Fig. 7) from a laser light source 64 (refer to Fig. 3) is emitted upward in an inclined direction in front of the tip part 36 as the auxiliary measurement light from the auxiliary measurement light exit window 62.

The laser light 66 emitted from the auxiliary measurement light exit window 62 can advance higher as the distance to the subject is increased. Thus, a height of an irradiation location of the laser light 66 captured in the captured image is increased as the distance to the subject is increased. By using this property, a distance to the irradiation location (the subject present at the irradiation location) is actually measured (measured) based on the irradiation location of the laser light 66 captured in the captured image in the endoscope system 10.

With reference to Fig. 1 again, the light source device 14 supplies the illumination light to the endoscope 12. The display 16 is, for example, a well-known liquid crystal display and displays the captured image captured by the endoscope 12 (image sensor 60), a result of image analysis performed by the processor device 20, and the like. The user interface 18 is an input device for performing input and the like into the processor device 20 and is a keyboard, a mouse, a foot pedal, a touch panel, a microphone, and/or a motion sensor or the like.

As illustrated in Fig. 3, the processor device 20 is provided with a program storage unit 70 and a central control unit 72. The program storage unit 70 stores a program related to various types of processing, control, or the like. The central control unit 72 functions as a pseudodistance data generation unit 74, an actual measurement distance data acquisition unit 76, and a distance data correction unit 78 by operating the program stored in the program storage unit 70.

The pseudodistance data generation unit 74 generates pseudodistance data obtained by estimating the distance to the subject for a plurality of locations within an imaging range of the image sensor 60 (pseudodistance data generation step). Specifically, a three-dimensional structure model of the subject is constructed using Structure-from-Motion (SfM). That is, the three-dimensional structure model of the subject is constructed using captured images of a plurality of frames captured by changing a relative positional relationship between the subject and the image sensor 60.

The constructed three-dimensional structure model, for example, represents the subject as a polygonal object (a set of polygonal shapes (for example, triangular shapes)) (refer to Fig. 4). The pseudodistance data generation unit 74 calculates the distance to the subject (a distance between the subject present at each location and the tip part 36) for the plurality of locations within the imaging range based on vertex coordinates of each surface (each polygonal shape) constituting the subject. The distance to the subject at each location calculated in this manner is the pseudodistance data according to the embodiment of the present invention.

By using the three-dimensional structure model of the subject (the vertex coordinates of each surface constituting the subject), the pseudodistance data can be generated (calculated) for any location within the imaging range. Thus, the location for generating (calculating) the pseudodistance data can be appropriately set. For example, the pseudodistance data may be generated (calculated) for all locations within the imaging range. In addition, the pseudodistance data may be generated (calculated) for only a part of the locations within the imaging range, for example, a location corresponding to a vertex of each surface constituting the subject. However, in correcting the pseudodistance data described later, the pseudodistance data at a location for which actual measurement distance data is acquired is used. Thus, the pseudodistance data generation unit 74 generates (calculates) the pseudodistance data for at least the location for which the actual measurement distance data is acquired.

The actual measurement distance data acquisition unit 76 acquires the actual measurement distance data obtained by actually measuring (measuring) the distance to the subject for at least one point within the imaging range (actual measurement distance data acquisition step). Specifically, the actual measurement distance data acquisition unit 76 analyzes the captured image, actually measures (measures) the distance to the irradiation location (the subject present at the irradiation location) based on the irradiation location of the auxiliary measurement light (laser light 66) captured in the captured image, and acquires the actual measurement distance data. Each time imaging is performed (once per frame), the actual measurement distance data acquisition unit 76 actually measures (measures) the distance to the irradiation location captured in the captured image and acquires the actual measurement distance data.

The distance data correction unit 78 corrects the pseudodistance data using the actual measurement distance data (distance data correction step). In the present embodiment, the distance data correction unit 78 divides the subject into a plurality of regions and performs correction (correction of the pseudodistance data using the actual measurement distance data) for each region. Hereinafter, a method of performing correction for each region obtained by dividing the subject into the plurality of regions will be specifically described using Fig. 4 to Fig. 8.

Hereinafter, a case where captured images P1, P2, and P3 illustrated in Fig. 5, Fig. 6, and Fig. 7, respectively, are acquired for the subject in which a protruding portion 82 of a square pyramid shape that is found in a lesion portion (tumor) and that has a left inclined surface 82L, a front inclined surface 82F, and a right inclined surface 82R is present inside (on a lower interior wall 80D) a tubular body 80 that is found in the gastrointestinal tract and that has a left interior wall 80L, the lower interior wall 80D, and a right interior wall 80R as illustrated in Fig. 4, by bending the bendable part 34 of the endoscope 12 right and left inside the subject (tubular body 80) to perform imaging in an order of imaging ranges E1, E2, and E3 will be described.

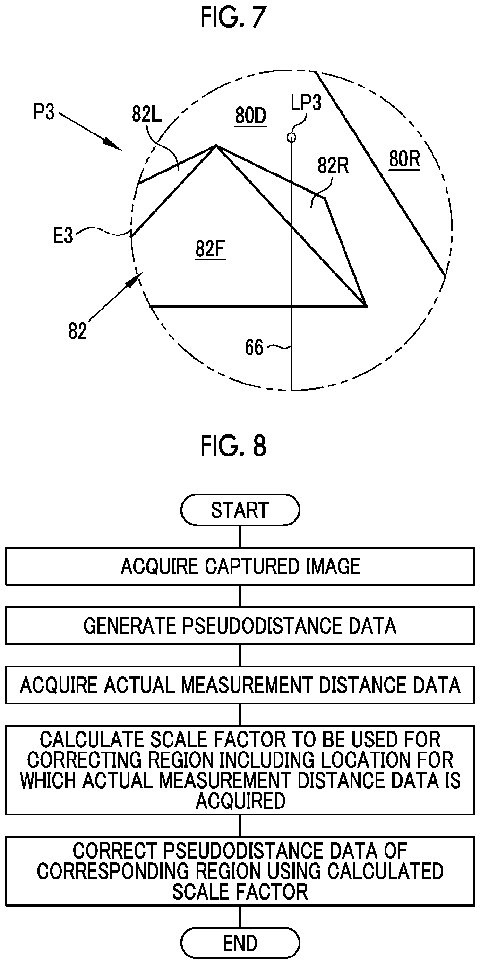

As illustrated in Fig. 8, in a case where the captured images P1, P2, and P3 (refer to Fig. 5, Fig. 6, and Fig. 7) are acquired by performing imaging in the order of the imaging ranges E1, E2, and E3 (refer to Fig. 4), the pseudodistance data generation unit 74 constructs a three-dimensional structure model of the inside of the tubular body 80 using the captured images P1, P2, and P3. In the constructed three-dimensional structure model, the protruding portion 82 having the left inclined surface 82L, the front inclined surface 82F, and the right inclined surface 82R is present inside (on the lower interior wall 80D) the tubular body 80 having the left interior wall 80L, the lower interior wall 80D, and the right interior wall 80R (refer to Fig. 4). The pseudodistance data generation unit 74 generates the pseudodistance data using the constructed three-dimensional structure model. In the present embodiment, the pseudodistance data is generated for all locations captured in the captured images P1, P2, and P3.

In addition, in a case where the captured images P1, P2, and P3 are acquired, the actual measurement distance data acquisition unit 76 acquires (measures) the actual measurement distance data. In the present example, a distance from the captured image P1 illustrated in Fig. 5 to an irradiation location LP1 (the irradiation location of the laser light 66) on the left interior wall 80L of the tubular body 80 is acquired (measured) as the actual measurement distance data. In addition, a distance from the captured image P2 illustrated in Fig. 6 to an irradiation location LP2 on the front inclined surface 82F of the protruding portion 82 is acquired as the actual measurement distance data. Furthermore, a distance from the captured image P3 illustrated in Fig. 7 to an irradiation location LP3 on the lower interior wall 80D of the tubular body 80 is acquired as the actual measurement distance data.

After generation of the pseudodistance data and acquisition of the actual measurement distance data are performed, the distance data correction unit 78, for a region including the location (the irradiation location of the laser light 66) at which the actual measurement distance data is acquired, calculates a scale factor for correcting the pseudodistance data of the region and corrects the pseudodistance data of the region using the calculated scale factor.

Specifically, for the irradiation location LP1 of the captured image P1 illustrated in Fig. 5, a scale factor (hereinafter, a first scale factor) for correcting the pseudodistance data of the left interior wall 80L that is a region including the irradiation location LP1 is calculated by dividing the actual measurement distance data of the irradiation location LP1 by the pseudodistance data. The pseudodistance data of the left interior wall 80L is corrected by multiplying each piece of the pseudodistance data constituting the left interior wall 80L by the first scale factor.

Similarly, for the irradiation location LP2 of the captured image P2 illustrated in Fig. 6, the distance data correction unit 78 calculates a scale factor (hereinafter, a second scale factor) for correcting the pseudodistance data of the front inclined surface 82F that is a region including the irradiation location LP2 by dividing the actual measurement distance data of the irradiation location LP2 by the pseudodistance data. The pseudodistance data of the front inclined surface 82F is corrected by multiplying each piece of the pseudodistance data constituting the front inclined surface 82F by the second scale factor.

Furthermore, for the irradiation location LP3 of the captured image P3 illustrated in Fig. 7, the distance data correction unit 78 calculates a scale factor (hereinafter, a third scale factor) for correcting the pseudodistance data of the lower interior wall 80D that is a region including the irradiation location LP3 by dividing the actual measurement distance data of the irradiation location LP3 by the pseudodistance data. The pseudodistance data of the lower interior wall 80D is corrected by multiplying each piece of the pseudodistance data constituting the lower interior wall 80D by the third scale factor.

As described above, for the region for which the actual measurement distance data is present, the distance data correction unit 78 corrects the pseudodistance data of the region using the scale factor calculated from the actual measurement distance data.

On the other hand, for the region such as the left inclined surface 82L and the right inclined surface 82R for which the actual measurement distance data is not present, the distance data correction unit 78 determines the scale factor to be used for correcting the region using the scale factor of the region for which the actual measurement distance data is present as the scale factor. Specifically, the pseudodistance data is corrected by calculating an average value of the scale factors (in the present embodiment, an average value of the first to third scale factors) of the regions for which the actual measurement distance data is present as the scale factor and multiplying each piece of the pseudodistance data constituting the region for which the actual measurement distance data is not present by the average value.

Correction of the region for which the actual measurement distance data is not present is not limited to the above method. For example, a region as a correction target may be corrected using the scale factor of a region closest to the region as the correction target among the regions for which the actual measurement distance data is present or the scale factor of a region having the longest length of contact with the region as the correction target among the regions for which the actual measurement distance data is present.

In addition, instead of the above average value, the region as the correction target may be corrected using a value obtained by calculating a weighted average of the scale factors of the regions for which the actual measurement distance data is present as the scale factor, considering an importance level of the region. In this case, it is considered to set the importance level to be higher as the region is closer to the region as the correction target or to set the importance level to be higher as the region has a longer length of contact with the region as the correction target.

In addition, it is considered that the actual measurement distance data is present for a plurality of locations within a common region. In this case, the scale factor for each location is calculated from the pseudodistance data and the actual measurement distance data of each location. Next, for any one location, the pseudodistance data of another location is corrected using the scale factor calculated from the location. Next, a difference between the pseudodistance data after correction and the actual measurement distance data of the corresponding location is obtained. The above processing may be performed for all locations, and the pseudodistance data of the region as the correction target may be corrected using the scale factor that results in the smallest difference.

In a case where the actual measurement distance data is present for the plurality of locations within the common region, correction may be performed using an average value of the scale factors for each location calculated from the pseudodistance data and the actual measurement distance data of each location as the scale factor. In addition, correction may be performed using a value obtained by calculating a weighted average of the scale factors for each location as the scale factor, considering the importance level. In this case, it is considered to set the importance level to be higher as the scale factor is calculated from the actual measurement distance data of a location closer to a center of the region as the correction target.

As described above, according to the endoscope system 10 according to the embodiment of the present invention, by correcting the pseudodistance data obtained by estimating the distance to the subject using the actual measurement distance data obtained by actually measuring the distance to the subject, the distance to the subject can be more accurately perceived, compared to that in a case where correction is not performed.

While an example of dividing the subject into regions based on the three-dimensional structure model of the subject constructed by the pseudodistance data generation unit 74 in dividing the subject into the plurality of regions has been described in the embodiment, a method of dividing the subject is not limited thereto and can be freely set. For example, the subject may be divided into concentric regions centered at one location on the subject. In addition, the subject may be divided into radial regions centered at one location on the subject. Furthermore, the subject may be divided into regions of a matrix by a plurality of parallel line segments extending horizontally and vertically.

In addition, while an example of performing correction for each region by dividing the subject into the plurality of regions has been described in the embodiment, correction may be performed without dividing the regions (using the entire subject as one region). In this case, the scale factor to be used for correction is determined using the same method as that in a case where the actual measurement distance data is present for the plurality of locations within the common region. The pseudodistance data of all locations may be corrected using the determined scale factor.

In addition, while an example of actually measuring (measuring) the distance from the captured image of one frame to one location of the subject by irradiating the subject with one beam of the laser light has been described in the embodiment, the present invention is not limited thereto. It may be configured to actually measure (measure) the distance from the captured image of one frame to each of the plurality of locations of the subject by irradiating the subject with a plurality of beams of the laser light.

Furthermore, while an example of acquiring (measuring) the actual measurement distance data by performing irradiation with the laser light as the auxiliary measurement light has been described in the embodiment, the actual measurement distance data may be acquired (measured) by performing irradiation with light other than the laser light as the auxiliary measurement light. In addition, while an example of acquiring the actual measurement distance data based on the irradiation location of the laser light has been described, the actual measurement distance data may be acquired (measured) using a time required from irradiation of the subject with an electromagnetic wave such as the laser light to reception of a reflected wave of the electromagnetic wave with which irradiation is performed. Of course, the actual measurement distance data may be acquired using a time required from emission of a sound wave or an ultrasound wave other than the electromagnetic wave to reception of the reflected wave.

Furthermore, an object of which a size is determined in advance (hereinafter, a size-determined object), such as a part of the tip part 36 or the forceps protruding into the body cavity from the outlet 56 of the forceps channel, is also captured in the captured image captured by the endoscope 12. Thus, a distance to a location near or adjacent to the size-determined object may be detected (measured) based on the size of the size-determined object captured in the captured image, and the detected (measured) distance may be acquired as the actual measurement distance data.

In addition, while an example in which the pseudodistance data generation unit 74 generates the pseudodistance data using the three-dimensional structure model of the subject constructed from the captured image of the plurality of frames captured by changing the relative positional relationship between the subject and the image sensor 60 has been described in the embodiment, the present invention is not limited thereto. The pseudodistance data generation unit 74 may be configured to generate the pseudodistance data using a distance estimation model that is generated by machine learning using the captured images of the plurality of frames captured by changing the relative positional relationship between the subject and the image sensor 60.

Furthermore, while an example in which the processor device 20 of the endoscope system 10 functions as the processor according to the embodiment of the present invention has been described in the embodiment, an image processing processor device may be provided separately from the endoscope system 10, and the image processing processor device may function as the processor according to the embodiment of the present invention. In this case, it may be configured to input the captured image captured by the endoscope 12 into the image processing processor device. In addition, it may be configured to generate the pseudodistance data, acquire the actual measurement distance data, and correct the pseudodistance data using the actual measurement distance data by causing a central control unit of the image processing processor device to function as the pseudodistance data generation unit 74, the actual measurement distance data acquisition unit 76, and the distance data correction unit 78 described above.

In the embodiment, a hardware structure of a processing unit that executes various types of processing of the central control unit 72, the pseudodistance data generation unit 74, the actual measurement distance data acquisition unit 76, the distance data correction unit 78, and the like includes various processors illustrated below. The various processors include a central processing unit (CPU) that is a general-purpose processor functioning as various processing units by executing software (program), a programmable logic device (PLD) such as a field programmable gate array (FPGA) that is a processor having a circuit configuration changeable after manufacture, a dedicated electric circuit that is a processor having a circuit configuration dedicatedly designed to execute various types of processing, and the like.

One processing unit may be composed of one of the various processors or may be composed of a combination of two or more processors of the same type or different types (for example, a plurality of FPGAs or a combination of a CPU and an FPGA). In addition, a plurality of processing units may be composed of one processor. As an example in which the plurality of processing units are composed of one processor, first, as represented by a computer such as a client and a server, a form in which one processor is composed of a combination of one or more CPUs and software and the processor functions as the plurality of processing units is possible. Second, as represented by a system on chip (SoC) and the like, a form of using a processor that implements functions of the entire system including the plurality of processing units in one integrated circuit (IC) chip is possible. Accordingly, various processing units are configured using one or more of the various processors as a hardware structure.

Furthermore, the hardware structure of the various processors is, more specifically, an electric circuit (circuitry) in the form of a combination of circuit elements such as semiconductor elements. In addition, a hardware structure of the storage unit is a storage device such as a hard disk drive (HDD) and a solid state drive (SSD).

### Explanation of References

10: endoscope system (medical endoscope system)
12: endoscope
14: light source device
16: display
18: user interface
20: processor device (processor)
30: insertion part
32: operating part
34: bendable part
36: tip part
38: inlet
40, 42, 44: rotary dial
46, 48, 50: push button
52: illumination window
54: observation window
56: outlet
58: air supply and water supply port
60: image sensor
62: auxiliary measurement light exit window
64: laser light source
66: laser light
70: program storage unit
72: central control unit
74: pseudodistance data generation unit
76: actual measurement distance data acquisition unit
78: distance data correction unit
80: tubular body
80L: left interior wall
80D: lower interior wall
80R: right interior wall
82: protruding portion
82L: left inclined surface
82F: front inclined surface
82R: right inclined surface
E1, E2, E3: imaging range
P1, P2, P3: captured image
LP1, LP2, LP3: irradiation location

## Claims

1. A medical endoscope system comprising:
an endoscope (12) including an image sensor (60) that acquires a captured image (P1, P2, P3) by imaging an inside of a body cavity as a subject; and
one or more processors (20) configured to:
generate pseudodistance data by estimating a distance to the subject for a plurality of locations within an imaging range of the image sensor using the captured image;
acquire actual measurement distance data by measuring the distance to the subject for at least one location within the imaging range; and
correct the pseudodistance data using the actual measurement distance data.

2. The medical endoscope system according to claim 1,
wherein the pseudodistance data is generated using a three-dimensional structure model of the subject that is constructed from a plurality of frames of the captured images obtained while changing a relative positional relationship between the subject and the image sensor (60).

3. The medical endoscope system according to claim 1,
wherein the pseudodistance data is generated using a distance estimation model that is generated by machine learning using a plurality of frames of the captured images obtained while changing a relative positional relationship between the subject and the image sensor (60).

4. The medical endoscope system according to claim 1,
wherein the actual measurement distance data is measured using the captured image obtained by irradiating the subject with auxiliary measurement light of which an optical axis is inclined with respect to an imaging optical axis of the image sensor (60).

5. The medical endoscope system according to claim 4,
wherein the auxiliary measurement light is laser light (66).

6. The medical endoscope system according to claim 5,
wherein the subject is a gastrointestinal tract.

7. The medical endoscope system according to any one of claims 1 to 6,
wherein the one or more processors (20) are configured to:
calculate a scale factor by dividing the actual measurement distance data by the pseudodistance data of the same location; and
correct the pseudodistance data of a different location by multiplying the pseudodistance data of the different location by the calculated scale factor.

8. The medical endoscope system according to claim 7,
wherein the one or more processors (20) are configured to correct the pseudodistance data of all locations by multiplying each piece of the pseudodistance data by the calculated scale factor.

9. The medical endoscope system according to claim 7,
wherein the one or more processors (20) are configured to:
divide the subject into a plurality of regions; and
correct the pseudodistance data of a region including the same location by multiplying the pseudodistance data within the region including the same location by the scale factor.

10. The medical endoscope system according to claim 9,
wherein the one or more processors (20) are configured to determine a scale factor for correcting a region lacking the actual measurement distance data using a scale factor for correcting a region with the actual measurement distance data.

11. The medical endoscope system according to claim 10,
wherein the one or more processors (20) are configured to correct the region lacking the actual measurement distance data using an average value of the scale factors used for correcting the regions with the actual measurement distance data, as the scale factor.

12. The medical endoscope system according to claim 9,
wherein the one or more processors (20) are configured to determine a scale factor to be used for correcting a region with a plurality of pieces of the actual measurement distance data using scale factors for each of these locations with the actual measurement distance data.

13. The medical endoscope system according to claim 12,
wherein the one or more processors (20) are configured to correct the region with the plurality of pieces of actual measurement distance data using an average value of the scale factors for each of these locations with the actual measurement distance data, as the scale factor.

14. An operation method of a medical endoscope system, the method comprising:
a pseudodistance data generation step of generating pseudodistance data by estimating a distance to a subject for a plurality of locations within an imaging range of an image sensor of an endoscope using a captured image captured by the image sensor;
an actual measurement distance data acquisition step of acquiring actual measurement distance data by measuring the distance to the subject for at least one location within the imaging range; and
a distance data correction step of correcting the pseudodistance data using the actual measurement distance data.
